# EUROPÄISCHE PATENTANMELDUNG

(11) **EP 2 730 641 A1**
(43) Veröffentlichungstag der Anmeldung: **14.05.2014**
(21) Anmeldenummer: 13190196.9
(22) Anmeldetag: 25.10.2013
(51) Int. Cl.: C12M 1/107, C12M 1/00, C12M 1/34, F17B 1/14, F16K 17/192

(54) **Biogasreaktor mit einer Überdruck-Unterdrucksicherung**

(30) Priorität: 12.11.2012 DE 102012110812
(71) Anmelder: 4Biogas GmbH & Co. KG, 44229 Dortmund (DE)
(72) Erfinder: Karl, Rainer, 99718 Clingen (DE); Kiomall, Daniel, 44137 Dortmund (DE); Bexten, Matthias, 33378 Rheda-Wiedenbrück (DE); Brachthäuser, Dr.Benno, 44229 Dortmund (DE)
(74) Vertreter: Tarvenkorn, Oliver

(57) **Zusammenfassung**

Die Erfindung betrifft einen Biogasreaktor (1) mit einer ein Gehäuse (18) aufweisenden Überdruck-Unterdrucksicherung (2). Um ein Nachfüllen der Überdruck-Unterdrucksicherung (2) mit Flüssigkeit und eine Verdampfung der Flüssigkeit zu vermeiden, schlägt die Erfindung vor, dass das Gehäuse (18) ein Dach (2a) aufweist, das auf seiner dem Gehäuseinneren (17) abgewandten Seite einen Flüssigkeitsaufnahmebereich (14) und im Bereich oberhalb des Gehäuseinneren (17) eine Öffnung (15) aufweist, wobei das Dach (2a) ein Fermenterdach (3) oder ein Teil des Fermenterdaches (3) ist.

## Beschreibung

Die Erfindung betrifft einen Biogasreaktor gemäß dem Oberbegriff des Anspruches 1.

Zudem betrifft die Erfindung eine Überdruck-Unterdrucksicherung sowie ein Verfahren zum Schutz vor Überdruck und Unterdruck in einem Fermenter eines Biogasreaktors.

Biogas gewinnt als Energieträger zunehmend an Bedeutung. Biogasreaktoren, d.h. Anlagen zur Gewinnung, Aufbereitung, Speicherung und/oder Nutzung von Biogas, weisen eine Überdruck-Unterdrucksicherung auf. Eine Überdruck-Unterdrucksicherung ist eine durch überhöhten Gasdruck ausgelöste Sicherheitseinrichtung, die den Fermenter eines Biogasreaktors vor zerstörendem Überdruck bei Überfüllung oder einer anderen Störung bewahrt bzw. den Fermenter eines Biogasreaktors durch Einlassen von Luft vor zerstörendem Unterdruck schützt.

Überdruck-Unterdrucksicherungen arbeiten häufig auf dem Prinzip des hydrolytischen Druckes. Hierzu ist an dem Gaslager bzw. an dem Fermenter ein Stutzen angebracht, über den das Gas im Falle eines Überdruckereignisses entweichen kann. An dem Stutzen ist dabei eine mit Wasser gefüllte Röhre befestigt, die an der Unterseite eine Öffnung besitzt, welche wiederum mit dem Gasstutzen verbunden ist. Je nachdem wie hoch die Wassersäule gefüllt ist, steht dem Gasdruck der jeweilige hydrolytische Druck der Wassersäule entgegen. Der hydrolytische Druck beträgt pro Meter Wassersäule 0,1 bar. Somit ist die Höhe der mit Wasser gefüllten Säule ausschlaggebend für die Druckfestigkeit der Überdruck-Unterdrucksicherung. Bei einer Wassersäule mit einer Höhe von 400 mm wird beispielsweise 40 mbar Druck benötigt, um das Wasser aus der Säule zu verdrängen. Erst nachdem das Wasser aus der Säule verdrängt worden ist, kann das Gas entweichen.

Aus den nach dem Prinzip des hydraulischen Druckes arbeitenden Überdruck-Unterdrucksicherungen ergibt sich jedoch die Problematik, dass nach Auslösen der Überdrucksicherung diese wieder mit Wasser gefüllt werden muss. Dies erfolgt entweder mit Hilfe eines Schwimmers, mit dessen Hilfe über eine Wasserleitung Wasser nachlaufen kann oder manuell. Die Installation einer Wasserleitung ist jedoch häufig nicht möglich. Auch ist das manuelle Nachfüllen häufig zu gefährlich und zu zeitintensiv oder ist schlichtweg nicht ausfallssicher genug. Eine weitere Problematik bei manuell nachfüllbaren Überdruck-Unterdrucksicherungen ist die mögliche Verdampfung der Flüssigkeit in wärmeren Jahreszeiten und Regionen. Hierbei kann es sehr schnell zu einem Ausfall der Überdruck-Unterdrucksicherung kommen, was den Verlust großer Mengen an Biogas zur Folge hat, was wiederum wirtschaftlich und emissionsrechtlich nicht wünschenswert ist.

Es ist deshalb Aufgabe der vorliegenden Erfindung, diese Nachteile zu vermeiden.

Diese Aufgabe wird mit den Merkmalen des Anspruches 1 gelöst.

Erfindungsgemäß weist das Gehäuse ein Dach auf, das auf seiner dem Gehäuseinneren abgewandten Seite einen Flüssigkeitsaufnahmebereich und im Bereich oberhalb des Gehäuseinneren eine Öffnung aufweist, wobei das Dach ein Fermenterdach oder ein Teil eines Fermenterdaches ist.

Die Überdruck-Unterdrucksicherung des erfindungsgemäßen Biogasreaktors weist, wie die meisten Überdruck-Unterdrucksicherungen, eine Flüssigkeitssäule auf, vorzugsweise eine Wassersäule, die füllhöhenabhängig für einen Gegendruck sorgt. Diese ist direkt unter dem Dach der Überdruck-Unterdrucksicherung installiert und wird kontinuierlich mit an dem Dach der Überdruck-Unterdrucksicherung aus Wasserdampf kondensiertem Wasser gefüllt. Tritt ein Überdruckereignis ein, so wird das in der Unterdrucksicherung befindliche Wasser in den Flüssigkeitsaufnahmebereich verdrängt und das in die Überdruck-Unterdrucksicherung eingetretene Gas kann wieder austreten. In einer hier verwendeten Terminologie wird als Flüssigkeitsaufnahmebereich ein die Flüssigkeit sammelnder Aufnahmebereich bezeichnet, wie beispielsweise eine Vertiefung in dem Fermenterdach. Der Flüssigkeitsaufnahmebereich kann vorzugsweise die Form einer Wanne oder eine seitliche Begrenzung aufweisen. Bei der seitlichen Begrenzung kann es sich wiederum um eine Art Manschette handeln. Ist das Überdruckereignis behoben, so kann sich die Überdruck-Unterdrucksicherung wieder mit Wasser füllen. Hierdurch wird vorteilhafterweise erreicht, dass ein übermäßiges Austreten von Biogas verhindert wird. Ein weiterer Vorteil ist es, dass die Überdruck-Unterdrucksicherung entgegen dem aufgezeigten Stand der Technik vollständig wartungsfrei ist.

Ein Fermenter einer Biogasreaktor weist häufig ein Mannloch auf. Eine vorteilhafte Ausgestaltung der Erfindung sieht daher vor, dass der Teil des Fermenterdaches ein Mannloch ist.

Eine praktikable Variante der Erfindung sieht vor, dass die Gehäusewand eine Öffnung aufweist, wobei die Öffnung eine von dem oberen Gehäuserand sich in das Innere der Gehäusewand erstreckende Öffnung ist. Hierdurch wird vorteilhafterweise erreicht, dass entgegen dem Stand der Technik die Überdruck-Unterdrucksicherung nicht regelmäßig mit Wasser gefüllt werden muss oder keine aufwändigen konstruktiven Ausgestaltungen, wie installierte Wasserleitungen, erforderlich sind, da durch die Öffnung in der Gehäusewand direkt auch Kondenswasser in das Innere der Überdruck-Unterdrucksicherung gelangen kann. Somit trägt auch die Öffnung in der Gehäusewand weiter zu einer vollständig wartungsfreien Überdruck-Unterdrucksicherung bei.

Die Erfindung sieht auch eine Überdruck-Unterdrucksicherung mit einem Gehäuse vor, das ein Dach aufweist, welches wiederum auf seiner dem Gehäuseinneren abgewandten Seite einen Flüssigkeitsaufnahmebereich und im Bereich oberhalb des Gehäuseinneren eine Öffnung aufweist.

Zudem sieht die Erfindung ein Verfahren zum Schutz vor Überdruck und Unterdruck in einem Fermenter eines Biogasreaktors vor, bei dem Flüssigkeit durch ein Gas aus einer Flüssigkeitssäule einer Überdruck-Unterdrucksicherung gedrängt wird. Hierbei wird die Flüssigkeit in einen die Flüssigkeit sammelnden Flüssigkeitsaufnahmebereich der Überdruck-Unterdrucksicherung gedrängt. Im Rahmen des erfindungsgemäßen Verfahrens befindet sich der Aufnahmebereich auf dem Dach eines Fermenters oder einem Teil eines Daches eines Fermenters. Nach dem Eindringen von Gas in die Überdruck-Unterdrucksicherung wird die Flüssigkeit vorzugsweise in den Aufnahmebereich gedrängt. Das in die Überdruck-Unterdrucksicherung gelangte Gas kann dabei aus einem Gasauslass der Überdruck-Unterdrucksicherung entweichen.

Im Folgenden wird die Erfindung anhand der Zeichnungen näher erläutert. Es zeigt in schematischer Darstellung:
- Fig. 1: in perspektivischer Ansicht einen Biogasreaktor gemäß der Erfindung,
- Fig. 2: in einer seitlichen Schnittansicht eine weitere Ausführungsform des Biogasreaktors aus Fig. 1 und
- Fig. 3a und 3b: in einer perspektivischen Ansicht die Außenansicht einer Über druck-Unterdrucksicherung gemäß der Erfindung und in einer seitlichen Schnittdarstellung das Gehäuseinnere der erfindungsgemäßen Überdruck-Unterdrucksicherung.

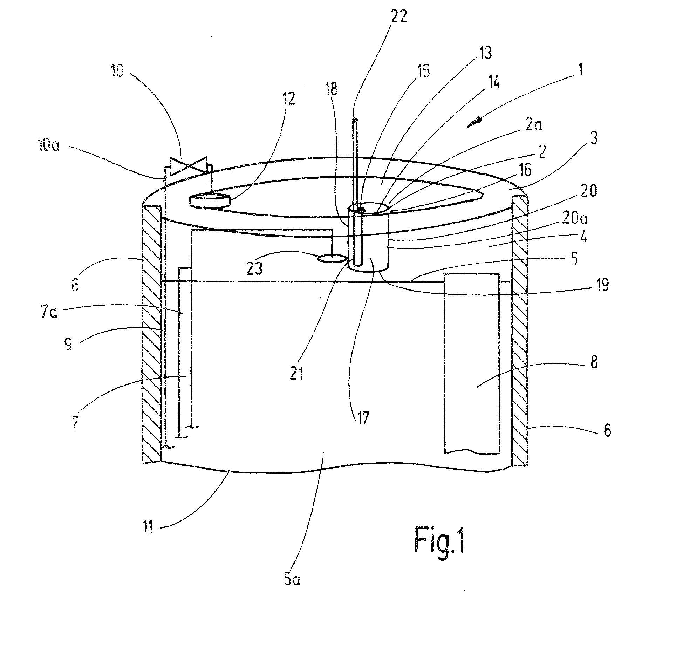

Fig. 1 zeigt einen erfindungsgemäßen Biogasreaktor, der mit dem Bezugszeichen 1 versehen ist.

Bei dem in Fig. 1 dargestellten Biogasreaktor 1 ist lediglich der obere Teil des Reaktors dargestellt. Der in dem deutschen Gebrauchsmuster Nr. 20 2011 106 472.0 offenbarte Biogasreaktor bildet die Grundlage für den in Fig. 1 gezeigten Biogasreaktor 1.

Eine wesentliche Komponente des Biogasreaktors 1 ist der Fermenter 11. Der Fermenter 11 ist mit dem Substrat 5a gefüllt. Oberhalb des Substratspiegels 5 befindet sich der Gasraum 4, der Bestandteil des Fermenters 11 ist. Der Fermenter 11 wird von der Wand 6 mit einer Isolierung 9 begrenzt. Oberseitig wird der Gasraum 4 bzw. der Fermenter 11 von dem Fermenterdach 3 begrenzt, das ein horizontales Bauteil ist und gleichzeitig den Biogasreaktor 1 bzw. den Fermenter 11 nach oben abschließt.

Weitere Komponenten in dem oberen Teil des Fermenters 11 sind der Gasdom 12, der Schieber 10 sowie die Gasleitung 10a. Das in dem Gasraum 4 produzierte Biogas wird an dem Gasdom 12 entnommen und über die Gasleitung 10a abgeführt. Der Gasfluss kann mittels des Schiebers 10, der oberhalb des Gasdoms 12 angeordnet ist, unterbrochen werden. Die Gasleitung 10a verläuft entlang der Wand 6 weiter durch den in Fig. 1 nicht gezeigten Boden des Fermenters 11. Parallel zur Gasleitung 10a in dem Gasraum 4 und in dem Fermenter 11 verläuft die Steigumwälzleitung 7. Die Steigumwälzleitung 7 mündet in dem oberen Teil des Biogasreaktors 1 in den Prallteller 23, so dass die Gülle auf das Substrat 5a verteilt wird. Die in den Prallteller 23 mündende Steigumwälzleitung 7 verläuft dabei innerhalb des Fermenters 11. Der Vorreaktorbehälter 8 erstreckt sich in dem oberen Teil des Biogasreaktors 1 und liegt in Form eines Rohres vor. Der Vorreaktorbehälter 8 erfüllt die Funktion, das aus dem unteren Teil des Biogasreaktors 1 in den Vorreaktorbehälter 8 gedrückte Substrat/Kosubstratgemisch weiter in den oberen Bereich des Biogasreaktors 1 zu drücken, damit es in den Fermenter 11 gelangt. Der in Fig. 1 gezeigte obere Teil des Biogasreaktors 1 weist zudem einen Teil einer Heizvorrichtung 7a auf, die innerhalb des Fermenters 11 angeordnet und der Steigumwälzleitung 7 zugeordnet ist. Eine fundierte Beschreibung der Heizvorrichtung 7a findet sich in dem deutschen Gebrauchsmuster Nr. 20 2011 106 472.0.

Unterhalb des Fermenterdaches 3 befindet sich, wie aus Fig. 1 weiter hervorgeht, das Gehäuse 18 der Überdruck-Unterdrucksicherung 2. Das Gehäuse 18 weist ein Dach 2a auf, das auf seiner dem Gehäuseinneren 17 abgewandten Seite einen Flüssigkeitsaufnahmebereich 14 und im Bereich oberhalb des Gehäuseinneren 17 eine Öffnung 15 aufweist. Als Dach 2a des Gehäuses 18 der Überdruck-Unterdrucksicherung 2 dient das Mannloch 13, so dass das Dach 2a Teil des Fermenterdaches 3 ist.

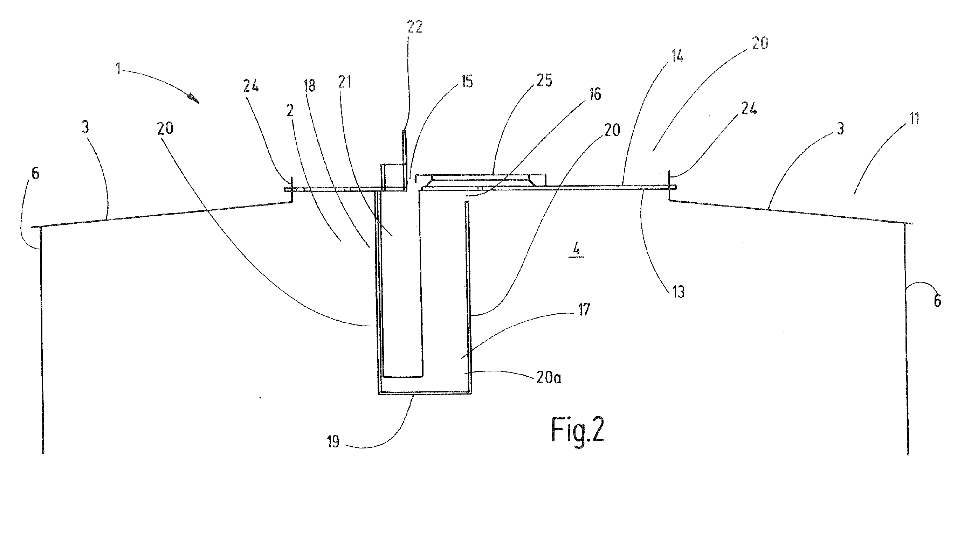

Die Überdruck-Unterdrucksicherung 2 weist, wie auch Fig. 2 verdeutlicht, eine Flüssigkeitssäule 21 auf, die sich bis zur Öffnung 15 erstreckt und an ihren beiden Endseiten offen ist. An ihrem unteren Ende schließt die Überdruck-Unterdrucksicherung 2 mit dem Gehäuseboden 19 ab. Der Gehäuseboden 19 liegt in Gestalt eines lösbaren Verschlussdeckels vor und ist zum unteren Ende der Flüssigkeitssäule 21 beabstandet angeordnet, so dass zwischen dem unteren Ende der Flüssigkeitssäule 21 und dem Gehäuseboden 19 ein Abstand ausgebildet ist.

Wie insbesondere weiter aus Fig. 2 hervorgeht, ist die Gehäusewand 20 mit einer Öffnung 16 versehen. Die Öffnung 16 ist eine von dem oberen Gehäuserand sich in das Innere der Gehäusewand 20 erstreckende Öffnung, durch die Gas aus dem Gasraum 4 in die Überdruck-Unterdrucksicherung 2 gelangen kann. Das durch die Öffnung 16 gelangte Gas verdrängt in der Überdruck-Unterdrucksicherung 2 befindliches Wasser. Das Wasser füllt in der Überdruck-Unterdrucksicherung 2 einen Teil des Bereiches aus, welcher unter anderem von dem Abstand definiert wird, der zwischen der Flüssigkeitssäule 21 und einem Teil der Gehäusewand 20, die die Form eines Gehäuserohres 20a hat, ausgebildet ist. Wie Fig. 2 zudem verdeutlicht, bildet das Mannloch 13 den Flüssigkeitsaufnahmebereich 14, an dem sich beidseitig das leicht abschüssige Fermenterdach 3 anschließt. Das den Flüssigaufnahmebereich 14 bildende Mannloch 13 weist eine seitliche Begrenzung 24 auf, die in Art einer Manschette vorliegt. Wie aus Fig. 2 weiterhin erkennbar ist, ist in das Mannloch 13 direkt das Schauglas 25 integriert, welches auch als Berstscheibe dient.

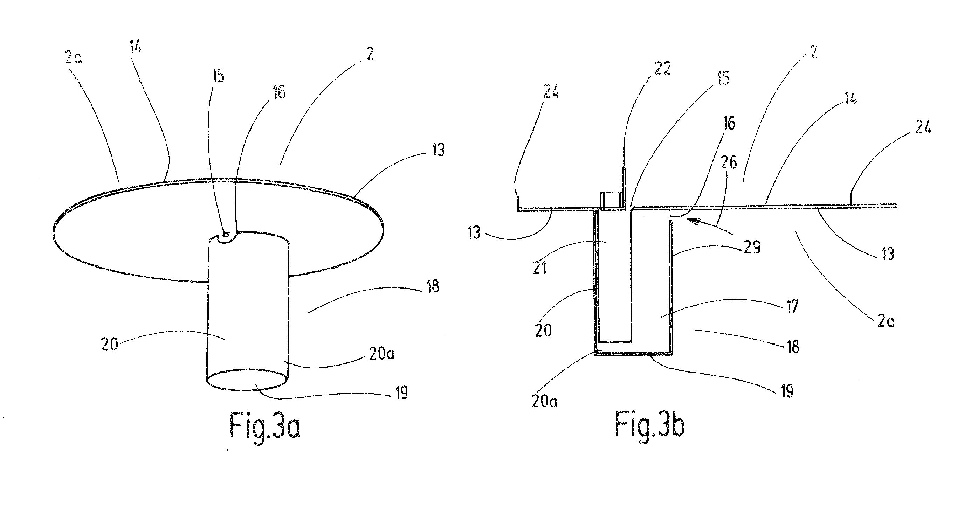

Die Fig. 3a und 3b zeigen, dass die Überdruck-Unterdrucksicherung 2 auch als separates Teil, d.h. losgelöst von dem in den Fig. 1 und 2 gezeigten Biogasreaktor 1, vorliegen kann. Als Dachvariante, bei der in den Fig. 3a und 3b gezeigten Überdruck-Unterdrucksicherung 2 dient wiederum das Mannloch 13, das auf seiner dem Gehäuseinneren 17 abgewandten einen Flüssigkeitsaufnahmebereich 14 und im Bereich oberhalb des Gehäuseinneren 17 die Öffnung 15 aufweist.

Wie aus Fig. 3a hervorgeht, ist die Überdruck-Unterdrucksicherung 2 unter dem Dach 2a gasdicht ausgestaltet. Der einzige Zugang in das Gehäuseinnere 17 ist die Öffnung 16. Tritt in dem in den Fig. 1 und 2 gezeigten Gasraum 4 ein Überdruck auf, so gelangt das Gas 26, wie Fig. 3b verdeutlicht, in das Gehäuseinnere 17 der Überdruck-Unterdrucksicherung 2 und drückt die in dem Gehäuseinneren 17 befindliche Flüssigkeit, d.h. das Wasser, von dem Gehäuserohr 20a durch die Flüssigkeitssäule 21 durch die Öffnung 15 in den Flüssigkeitsaufnahmebereich 14. Das Gas 26 kann dabei solange durch den Gasauslass 22 entweichen bis die Rücklaufkraft des Wassers in den Flüssigkeitsaufnahmebereich 14 größer als der Gasdruck ist. Danach füllt sich die Überdruck-Unterdrucksicherung 2 wieder mit Wasser und ist betriebsbereit.

Die vorliegende Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch machen. Beispielsweise kann die Öffnung 16 versetzt in der Gehäusewand 20 angeordnet sein.

### Bezugszeichenliste:

- 1: Biogasreaktor
- 2: Überdruck-Unterdrucksicherung
- 2a: Dach
- 3: Fermenterdach
- 4: Gasraum
- 5: Substratspiegel
- 5a: Substrat
- 6: Wand
- 7: Steigumwälzleitung
- 7a: Heizvorrichtung
- 8: Vorreaktorbehälter
- 9: Isolierung
- 10: Schieber
- 10a: Gasleitung
- 11: Fermenter
- 12: Gasdom
- 13: Mannloch
- 14: Flüssigkeitsaufnahmebereich
- 15, 16: Öffnung
- 17: Gehäuseinnere
- 18: Gehäuse
- 19: Gehäuseboden
- 20: Gehäusewand
- 20a: Gehäuserohr
- 21: Flüssigkeitssäule
- 22: Gasauslass
- 23: Prallteller
- 24: Begrenzung
- 25: Schauglas
- 26: Gas

## Patentansprüche

1. Biogasreaktor (1) mit einer ein Gehäuse (18) aufweisenden Überdruck-Unterdrucksicherung (2),
**dadurch gekennzeichnet,**
**dass** das Gehäuse (18) ein Dach (2a) aufweist, das auf seiner dem Gehäuseinneren (17) abgewandten Seite einen Flüssigkeitsaufnahmebereich (14) und im Bereich oberhalb des Gehäuseinneren (17) eine Öffnung (15) aufweist, wobei das Dach (2a) ein Fermenterdach (3) oder ein Teil eines Fermenterdaches (3) ist.

2. Reaktor nach Anspruch 1,
**dadurch gekennzeichnet,**
**dass** der Teil des Fermenterdaches (3) ein Mannloch (13) ist.

3. Reaktor nach Anspruch 1 oder 2,
**dadurch gekennzeichnet,**
**dass** die Überdruck-Unterdrucksicherung (2) eine Flüssigkeitssäule (21) aufweist, die sich bis zur Öffnung (15) erstreckt und an ihren beiden Endseiten offen ist und/oder die Überdruck-Unterdrucksicherung (2) an ihrem unteren Ende mit einem Gehäuseboden (19) abschließt.

4. Reaktor nach Anspruch 3,
**dadurch gekennzeichnet,**
**dass** der Gehäuseboden (19) in Form eines lösbaren Verschlussdeckels vorliegt und/oder zwischen dem unteren Ende der Flüssigkeitssäule (21) und dem Gehäuseboden (19) ein Abstand ausgebildet ist.

5. Reaktor nach einem der vorhergehenden Ansprüche,
**dadurch gekennzeichnet,**
**dass** die Wand (20) des Gehäuses (18) eine Öffnung (16) aufweist, wobei die Öffnung (16) eine von dem oberen Gehäuserand sich in das Innere der Gehäusewand (20) erstreckende Öffnung ist.

6. Reaktor nach einem der vorhergehenden Ansprüche,
**dadurch gekennzeichnet,**
**dass** der Flüssigkeitsaufnahmebereich (14) die Form einer Wanne aufweist und/oder der Flüssigkeitsaufnahmebereich (14) eine seitliche Begrenzung (24) aufweist.

7. Reaktor nach einem der Ansprüche 3 bis 6,
**dadurch gekennzeichnet,**
**dass** die Gehäusewand (20) die Form eines Rohres (20a) hat und zwischen Flüssigkeitssäule (21) und Gehäusewand (20) oder zwischen der Flüssigkeitssäule (21) und einem Teil der Gehäusewand (20) ein Abstand ausgebildet ist.

8. Überdruck-Unterdrucksicherung (2) mit einem Gehäuse (18),
**dadurch gekennzeichnet,**
**dass** das Gehäuse (18) ein Dach (2a) aufweist, das auf seiner dem Gehäuseinneren (17) abgewandten Seite einen Flüssigkeitsaufnahmebereich (14) und im Bereich oberhalb des Gehäuseinneren (17) eine Öffnung (15) aufweist.

9. Sicherung nach Anspruch 8,
**dadurch gekennzeichnet,**
**dass** in dem Gehäuseinneren (17) eine Flüssigkeitssäule (21) angeordnet ist, die sich bis zur Öffnung (15) erstreckt und an ihren beiden Endseiten offen ist und/oder der Flüssigkeitsaufnahmebereich (14) die Form einer Wanne hat, wobei der Flüssigkeitsaufnahmebereich (14) eine seitliche Begrenzung (24) aufweist.

10. Sicherung nach Anspruch 8 oder 9,
**dadurch gekennzeichnet,**
**dass** das Gehäuse (18) einen Gehäuseboden (19) aufweist, der in Form eines lösbaren Verschlussdeckels vorliegt.

11. Sicherung nach einem der Ansprüche 8 bis 10,
**dadurch gekennzeichnet,**
**dass** die Wand (20) des Gehäuses (18) eine Öffnung (16) aufweist, wobei die Öffnung (16) eine von dem oberen Gehäuserand sich in das Innere der Gehäusewand (20) erstreckende Öffnung ist.

12. Sicherung nach einem der Ansprüche 8 bis 11,
**dadurch gekennzeichnet,**
**dass** zwischen Flüssigkeitssäule (21) und Gehäusewand (20) oder zwischen Flüssigkeitssäule (21) und einem Teil der Gehäusewand (20) ein Abstand ausgebildet ist und/oder das Dach (2a) ein Fermenterdach (3) oder ein Mannloch (13) eines Fermenters (11) eines Biogasreaktors (1) ist.

13. Verfahren zum Schutz vor Überdruck und Unterdruck in einem Fermenter (11) eines Biogasreaktors (1), bei dem Flüssigkeit durch ein Gas (26) aus einer Flüssigkeitssäule (21) einer Überdruck-Unterdrucksicherung (2) gedrängt wird,
**dadurch gekennzeichnet,**
**dass** die Flüssigkeit in einen die Flüssigkeit sammelnden Flüssigkeitsaufnahmebereich (14) der Überdruck-Unterdrucksicherung (2) gedrängt wird.

14. Verfahren nach Anspruch 13,
**dadurch gekennzeichnet,**
**dass** sich der Flüssigkeitsaufnahmebereich (14) auf dem Dach (2a) eines Fermenters (11) oder einem Teil eines Daches (2a) eines Fermenters (11) befindet und/oder bei einem Eindringen von Gas (26) in die Überdruck-Unterdrucksicherung (2) die Flüssigkeit in den Aufnahmebereich (14) gedrängt wird.

15. Verfahren nach Anspruch 13 oder 14,
**dadurch gekennzeichnet,**
**dass** das in die Überdruck-Unterdrucksicherung (2) gelangte Gas (26) aus einem Gasauslass (22) der Überdruck-Unterdrucksicherung (2) entweicht und/oder das Gas (26) durch eine in einer Gehäusewand (20) der Überdruck-Unterdrucksicherung (2) befindliche Öffnung (16) in das Innere eines Gehäuses (18) der Überdruck-Unterdrucksicherung (2) gelangt.
